# EUROPÄISCHE PATENTANMELDUNG

(11) **EP 0 592 784 A1**
(43) Veröffentlichungstag der Anmeldung: **20.04.1994**
(21) Anmeldenummer: 93112688.2
(22) Anmeldetag: 07.08.1993
(51) Int. Cl.: C07H 15/04

(54) **Verfahren zur Aufarbeitung von fettalkoholischen Alkylpoly-glycosid-Lösungen**

(30) Priorität: 10.10.1992 DE 4234241
(71) Anmelder: HÜLS AKTIENGESELLSCHAFT, D-45764 Marl (DE)
(72) Erfinder: Schmidt, Stefan, Dr., D-45721 Haltern (DE)

(57) **Zusammenfassung**

Die bei der Herstellung der Alkylpolyglycoside zunächst anfallenden fettalkoholischen Alkylpolyglycosid-Lösungen werden einer Alkalibehandlung und danach einer Vakuumdestillation unterworfen. Dabei werden die zunächst noch sauren Lösungen mit Alkali auf einen pH-Wert von 10 bis 14 eingestellt, worauf sie mindestens 2 Stunden bei 60 bis 120 °C temperiert werden. Danach wird der Fettalkohol bei einem verminderten pH-Wert von 7 bis 9 im Vakuum abdestilliert.

## Beschreibung

Die Erfindung betrifft ein Verfahren zur Aufarbeitung von fettalkoholischen Alkylpolyglycosid-Lösungen, bei denen die Alkylgruppen der Alkylpolyglycoside und der Alkohole 8 bis 24 C-Atome aufweisen, durch Alkalibehandlung und Destillation.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylgruppen werden im allgemeinen durch ein- oder mehrstufige Synthesen hergestellt.

Ein einstufiges Herstellverfahren wird u. a. in DE-A-41 01 252 beschrieben.

Ein zweistufiges Verfahren wird beispielsweise in EP-A-0 306 652 angegeben, wonach man zunächst durch Glycosidierung mit n-Butanol ein n-Butylglycosid und darauf durch Umglycosidierung mit einem langkettigen Alkohol das gewünschte langkettige Alkylpolyglycosid herstellt.

Nach beendeter Reaktion liegen die Alkylpolyglycoside in langkettigen Alkoholen gelöst vor. Anschließend müssen diese Alkohole abgetrennt werden, wenn man in Wasser klar lösliche Produkte erhalten will.

In EP-A-0 387 912 wird ein einstufiges Herstellverfahren beschreiben, bei dem die langkettigen Alkohole zunächst mit Hilfe von Alkali gereinigt werden, bevor man sie mit Zucker säurekatalysiert umsetzt. Nach Neutralisation und Destillation des überschüssigen Fettalkohols werden Alkylglycoside mit verbesserter Farbe erhalten. Eine Alkalibehandlung der fettalkoholischen Alkylglycosid-Lösung erfolgt hier nicht.

Nach EP-A-0 132 046 wird die Reaktionsmischung statt mit wäßrigem Alkylhydroxid mit einer organischen Base, beispielsweise mit Natriumethylat, neutralisiert, wobei ein pH-Wert von 6,6 bis 7 eingestellt wird. Nach der Destillation wird ein farblich verbessertes Produkt erhalten. Eine Alkalibehandlung wird hier nicht vorgenommen.

Auch in EP-A-0 362 671 werden Alkylglycoside mit langkettigen Alkylgruppen in einem einstufigen Verfahren hergestellt. Nach der Reaktion werden hier die fettalkoholischen Alkylglycosid-Lösungen auf ca. 90 °C abgekühlt, worauf mit Alkali auf mindestens pH 8, vorzugsweise auf pH 8 bis 10 eingestellt wird. Anschließend oder nach 30 Minuten (Beispiele) werden die Alkohole aus der alkalischen Lösung destilliert. Nach abschließender Bleichung werden hellfarbige und farbstabile Alkylglycoside erhalten.

Nach diesem Verfahren werden, vor allem wenn mit Alkalihydroxiden alkalisch eingestellt wird, farblich noch nicht voll befriedigende Alkylpolyglycoside erhalten.

Aufgabe der vorliegenden Erfindung ist es daher, die Farbe und die Farbstabilität der Alkylpolyglycoside weiter zu verbessern.

Die Aufgabe wird erfindungsgemäß dadurch gelöst, daß man die fettalkoholischen Alkylpolyglycosid-Lösungen nach beendeter Reaktion zunächst auf einen pH-Wert von 10 bis 14 einstellt und dann für mindestens 2 Stunden bei 60 bis 120 °C temperiert, bevor der Fettalkohol bei einem niedrigeren pH-Wert von 7 bis 9 im Vakuum abdestilliert wird.

Die hier eingesetzten Alkylpolyglycoside können durch kontinuierliche oder diskontinuierliche, ein- oder mehrstufige Reaktion erhalten werden. Vorzugsweise wendet man das erfindungsgemäße Verfahren auf einstufig hergestellte Alkylpolyglycoside an.

Bei der Herstellung der Alkylpolyglycoside können als Zucker beispielsweise Glucose, Mannose, Galactose oder Talose eingesetzt werden, wobei Glucose bevorzugt verwendet wird. Geeignete langkettige Alkohole, die auch als Fettalkohole bezeichnet werden, sind beispielsweise Octanol, Decanol, Dodecanol, Tetradecanol und Hexadecanol. Die Alkylgruppen der Alkylpolyglycoside und die der Alkohole weisen vorzugsweise 10 bis 18 C-Atome und ganz besonders bevorzugt 12 bis 16 C-Atome auf.

Die Alkylpolyglycosid-Herstellung kann durch saure Katalysatoren, wie zum Beispiel Mineralsäuren, aliphatische und aromatische Sulfonsäuren oder durch saure Ionenaustauscher katalysiert werden.

Die Alkylpolyglycoside weisen im allgemeinen einen Polymerisationsgrad von 1 bis 5 auf, wobei Produkte mit Polymerisationsgraden von 1,1 bis 1,4 bevorzugt werden.

Zur Einstellung der fettalkoholischen Alkylpolyglycosid-Lösungen auf einen alkalischen pH-Wert können wäßrige oder alkoholische Lösungen von Alkali- oder Erdalkalihydroxiden oder Alkalialkoholate in Alkoholen eingesetzt werden. Die Alkalizugabe erfolgt dabei vorzugsweise bei der Temperatur, bei der die Alkylpolyglycoside hergestellt worden sind, oder bei der Temperatur, bei der die Lösungen temperiert werden sollen, oder bei einer Temperatur dazwischen.

Vorzugsweise wird mit wäßrigen Alkalihydroxiden alkalisch eingestellt. Beim Einsatz nichtwäßriger Lösungen wird der pH-Wert an mit Wasser verdünnten Proben ermittelt.

Bei sehr hohen pH-Werten kann es während der Alkalizugabe zu Ausfällungen kommen, die die Pumpfähigkeit der Lösungen beeinträchtigen. Bei zu niedrigen pH-Werten ist der Effekt für die Farbe der Produkte zu gering. Vorzugsweise wird ein pH-Wert von 10 bis 12 eingestellt.

Die Temperierung erfolgt meist in 3 bis 48 Stunden. Sie wird vorzugsweise nach 5 bis 30 Stunden abgeschlossen. Dabei wird bevorzugt eine Temperatur von 60 bis 80 °C eingestellt. Beim Temperieren kann man die Lösung rühren, schütteln oder einfach stehen lassen.

Nach der Temperierung werden die Alkohole vorzugsweise in einem Fallfilm- oder Dünnschichtverdampfer oder in einer Kombination aus beiden Geräten abdestilliert.

Zur weiteren Aufarbeitung werden die Alkylpolyglycoside in bekannter Weise in Wasser gelöst und anschließend gebleicht.

Die resultierenden Produkte zeichnen sich durch eine sehr gute Farbe und Farbstabilität aus. Der pH-Wert von wäßrigen Lösungen dieser Alkylpolyglycoside ist stabil.

Die folgenden Beispiele sollen die Erfindung verdeutlichen.

### Vergleichsbeispiel A

In einem 250-l-VA-Rührkessel werden
169 kg (860 Mol) Fettalkohol (67 % n-Dodecanol, 27 % n-Tetradecanol, 6 % n-Hexadecanol),
31 kg (172 Mol) wasserfreie Dextrose und
1,57 kg ( 8,26 Mol) p-Toluolsulfonsäure-monohydrat
in 6 Stunden bei 100 °C/15 hPa umgesetzt. Dazu wird zunächst das p-Toluolsulfonsäure-monohydrat im Fettalkohol gelöst und anschließend die wasserfreie Dextrose zugegeben. Das Reaktionswasser wird in einer Kühlfalle aufgefangen. Die erhaltene fettalkoholische Alkylpolyglycosid-Lösung wird auf 60 °C gekühlt und mit 25%iger wäßriger NaOH auf pH 11 eingestellt. Zur Bestimmung des pH-Wertes mit Hilfe einer Glaselektrode werden dabei Proben mit Isopropanol und Wasser im Gewichtsverhältnis 1 : 3 : 2 verdünnt.

Nach Filtration wird die Lösung über eine Kaskade von 3 Dünnschichtverdampfern geleitet (Dosierung: 5 1/h, DSV 1: 100 °C/100 hPa, DSV 2 und 3: 180 °C/1 hPa). Nach kurzer Zeit kommt es zu massiven Aufladungen und Vercrackungen, so daß die Destillation des Fettalkohols abgebrochen werden muß.

### Vergleichsbeispiel B

In dem Kessel von Vergleichsbeispiel A werden
147,4 kg (750 Mol) Fettalkohol (67 % n-Dodecanol, 27 % n-Tetradecanol, 6 % n-Hexadecanol),
18 kg (100 Mol) wasserfreie Glucose,
520 g ( 5,1 Mol) konz. Schwefelsäure und
450 g ( 4,25 Mol) Natriumhypophosphit
umgesetzt und dann aufgearbeitet. Dabei wird wie in Vergleichsbeispiel A verfahren. Auch hier gelingt die Abtrennung des Fettalkohols in der Dünnschichtverdampferkaskade nicht, da sich Zersetzungsprodukte in erheblichem Ausmaße bilden.

### Beispiel 1

In dem Kessel von Vergleichsbeispiel A werden
147,4 kg (750 Mol) Fettalkohol (67 % n-Dodecanol, 27 % n-Tetradecanol, 6 % n-Hexadecanol),
18 kg (100 Mol) wasserfreie Glucose und
1,29 kg ( 6,8 Mol) p-Toluolsulfonsäure-monohydrat
unter den Bedingungen von Vergleichsbeispiel A umgesetzt. Die Reaktionsmischung wird auf 70 °C abgekühlt und mit 25%iger wäßriger NaOH auf pH 11 gebracht.

Die Lösung wird dann 24 Stunden bei 70 °C temperiert. Danach wird die inzwischen schwach sauer reagierende Lösung mit 25%iger wäßriger NaOH auf pH 9 eingestellt. Eine Probe dieser fettalkoholischen Alkylpolyglycosid-Lösung wird bei 60 °C gelagert. Auch nach 12 Stunden liegt der pH-Wert unverändert bei 9.

Nach Filtration wird der Fettalkohol in der Dünnschichtverdampferkaskade problemlos abdestilliert. Das verbleibende Alkylpolyglycosid hat einen Restalkoholgehalt von 2 %. Das Produkt wird in Wasser gelöst und bei 80 °C und pH 7 mit 3,5 % Wasserstoffperoxid (bezogen auf das Alkylpolyglycosid) versetzt. Die Lösung wird 6 Stunden bei 80 °C gerührt. Die Wassermenge ist dabei so gewählt, daß nach Abschluß des Bleichvorgangs eine 50%ige wäßrige Lösung resultiert. Die Iodfarbzahl dieser Lösung liegt bei 7.

### Vergleichsbeispiel C (gemäß EP 0 362 671)

In einem 4-l-Rührkolben werden
2 364 g (12 Mol) Fettalkohol (67 % n-Dodecanol, 27 % Tetradecanol, 6 % n-Hexadecanol),
720 g ( 4 Mol) wasserfreie Dextrose und
4,96 g (26 mMol) p-Toluolsulfonsäure-monohydrat
in 4 Stunden bei 100 °C/20 hPa miteinander umgesetzt. Dabei wird vor der eigentlichen Reaktion zunächst eine Lösung von p-Toluolsulfonsäure in Fettalkohol hergestellt, bevor Dextrose zugegeben wird. Nach Beendigung der Reaktion wird bei 70 °C mit 25%iger wäßriger NaOH auf pH 11 eingestellt (pH-Kontrolle wie in Vergleichsbeispiel A). Anschließend wird der Fettalkohol sogleich am Rotationsverdampfer bei 180 °C/1 hPa so weit abdestilliert, daß ein Feststoff mit einem Restfettalkoholgehalt von < 2 % resultiert.

Das Produkt wird wie in Beispiel 1 angegeben gebleicht. Man erhält dabei eine 50%ige Lösung mit einer Iodfarbzahl von 110.

### Beispiel 2

In einem 4-l-Rührkolben werden
1 970 g (10 Mol) Fettalkohol (67 % n-Dodecanol, 27 % Tetradecanol, 6 % n-Hexadecanol),
240 g ( 1,3 Mol) wasserfreie Dextrose und
17,2 g (90 mMol) p-Toluolsulfonsäure-monohydrat
in 4 Stunden bei 100 °C/20 hPa miteinander umgesetzt. Auch hier wird vor der eigentlichen Reaktion zunächst eine Lösung von p-Toluolsulfonsäure in Fettalkohol hergestellt, bevor Dextrose zugesetzt wird. Nach Beendigung der Reaktion wird bei 70 °C mit 25%iger wäßriger NaOH auf pH 11 eingestellt.

Desweiteren wird wie in Beispiel 1 verfahren, wobei der Fettalkohol jedoch in einem Rotationsverdampfer bei 180 °C/1 hPa abdestilliert wird. Nach der Bleichung wird eine Iodfarbzahl von 15 erreicht.

## Patentansprüche

1. Verfahren zur Aufarbeitung saurer Lösungen von Alkylpolyglycosiden in Alkoholen, wobei die Alkylgruppen der Alkylpolyglycoside und Alkohole 8 bis 24 C-Atome aufweisen, durch Alkalibehandlung und Destillation,
dadurch gekennzeichnet,
daß man die Lösungen auf einen pH-Wert von 10 bis 14 einstellt, sie mindestens 2 Stunden bei 60 bis 120 °C temperiert und dann die Alkohole bei einem pH-Wert von 7 bis 9 im Vakuum destilliert.

2. Verfahren nach Anspruch 1,
dadurch gekennzeichnet,
daß die Alkylgruppen 10 bis 18 C-Atome aufweisen.

3. Verfahren nach Anspruch 2,
dadurch gekennzeichnet,
daß die Alkylgruppen 12 bis 16 C-Atome enthalten.

4. Verfahren nach Anspruch 1,
dadurch gekennzeichnet,
daß man die Lösungen auf einen pH-Wert von 10 bis 12 einstellt.

5. Verfahren nach Anspruch 1,
dadurch gekennzeichnet,
daß man 3 bis 48 Stunden temperiert.

6. Verfahren nach Anspruch 1,
dadurch gekennzeichnet,
daß man die Lösungen bei 60 bis 80 °C temperiert.
